(19)

**Europäisches Patentamt**
**European Patent Office**
**Office européen des brevets**

(11)  **EP 3 831 286 A1**

(12)  **EUROPEAN PATENT APPLICATION**

(43) Date of publication:
**09.06.2021 Bulletin 2021/23**

(21) Application number: **19306597.6**

(22) Date of filing: **06.12.2019**

(51) Int Cl.:
*A61B 5/021* (2006.01)          *A61B 5/024* (2006.01)
*A61B 5/08* (2006.01)           *A61M 16/10* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicants:
- **École Normale Supérieure Paris-Saclay**
  **94230 Cachan (FR)**
- **Etat Français - Ministère de la Défense - Direction centrale du service de santé des armées**
  **75614 Paris Cedex 12 (FR)**
- **Centre National de la Recherche Scientifique**
  **75016 Paris (FR)**
- **UNIVERSITE PARIS DESCARTES**
  **75006 Paris (FR)**

(72) Inventors:
- **AUDIFFREN, Julien**
  **1700 Fribourg (CH)**
- **LABOURDETTE, Christophe**
  **75013 PARIS (FR)**
- **VAYATIS, Nicolas**
  **75005 PARIS (FR)**
- **VIDAL, Pierre-Paul**
  **75005 PARIS (FR)**
- **HUMBERT, Pierre**
  **75006 PARIS (FR)**
- **DUBOST, Clément**
  **75014 PARIS (FR)**

(74) Representative: **A.P.I. Conseil**
**Immeuble Newton**
**4, rue Jules Ferry**
**64000 Pau (FR)**

(54)  **SYSTEM AND METHOD FOR PREDICTING DEPTH OF ANESTHESIA**

(57) The invention relates to a system (1) and method for predicting depth of anesthesia of a patient to obtain a representative value of said depth of anesthesia of the patient, comprising an acquisition mean and a communication mean configured for receiving the data, a storage module (30) on which are recorded data from a plurality of patient having undergone anesthesia, of at least four physiological variables, said physiological variables comprising: Heart Rate, Mean Blood Pressure, Respiratory Rate, and quantity of anesthetics agent, and a data processing module (40) connected to the storage module (30), said data processing module (40) being configured for predicting the depth of anesthesia, for example to adapt the dosage of anesthetic agent, to evaluate over-dosage of anesthetic agent and to deliver a finely tuned anesthesia dose especially for frail patients.

Figure 7

EP 3 831 286 A1

**Description**

**FILED OF THE INVENTION**

The present invention relates to the field of the prediction and optimization of the depth of anesthesia (DoA) of a patient under general anesthesia. The present invention relates to a system and method for predicting DoA of a patient to obtain a representative value of said DoA of the patient, comprising an acquisition mean and a communication mean configured for receiving the data, storage module on which are recorded data from a plurality of patient having undergone anesthesia, of at least four physiological variables, said physiological variables comprising: Heart Rate, Mean Blood Pressure, Respiratory Rate, and quantity of anesthetics agent, and a data processing module connected to the storage module, said data processing module being configured for predicting the DoA, for example to adapt the dosage of anesthetic agent, to prevent over-dosage of anesthetic agent and deliver a finely tuned anesthesia dose especially for frail patients. According to the present invention, the physiological variables recorded are the Heart Rate, the Mean Blood Pressure, the Respiratory Rate and the quantity of anesthetics agent.

**BACKGROUND OF THE INVENTION**

The three commonly admitted goals of anesthesia are lack of experience of surgery, nociceptive blockade and immobility for the needs of surgery. DoA has been defined by community as "the probability of non-response to stimulation, calibrated against the strength of the stimulus, the difficulty of suppressing the response, and the drug-induced probability of non-responsiveness at defined effect site concentrations". When consciousness does not disappear totally, the patient may be aware during the intervention and eventually remember memories of the surgery. This condition, named "intraoperative awareness with explicit recall", however rare, may cause post-operative stress disorder.

Assessing the DoA is a daily challenge for anesthesiologists. A few brain monitors based on electroencephalogram (EEG) analysis have been developed but they all have limitations. Consequently, the best assessment of the DoA is commonly thought to be the one made by the doctor in charge of the patient. This evaluation is based on the integration of several parameters including epidemiological, pharmacological and physiological data. However, it remains a tricky task and the burden of work in the theater makes it hard, if not impossible, to have an anesthesiologist dedicated to each room.

In that context, the gold-standard to quantify brain activity, during anesthesia, remains the electroencephalogram (EEG). Hence, several monitoring systems using EEG-based index have been proposed for DoA assessment, but they all have limitations (Bruhn et al., 2006). Besides, no point-of-care gold standard monitoring prevails for the DoA. The most used index is the Bispectral Index, (BIS, Covidien, Boulder, CO) proposed in the 2000's by an American company as a measure of the effect of anesthetics on the brain (Avidan et al., 2008; Kissin, 2000). The BIS provides a numerical value from 0 to 100 (no cerebral activity to awake and responsive) allowing for more accurate tailoring of the anesthetics administered. The usual numerical value for a surgical anesthesia lies between 40 and 60, whereas values < 40 indicate over-dosage of anesthetics and values >80 lack of anesthetics. Despite being largely used, mostly in the US, the BIS monitor presents several drawbacks such as high interindividual variability (Whitlock et al., 2011)(Avidan et al., 2008), low performance with volatile anesthetics (George Mychaskiw et al., 2001), and long latency and interferences with surgical knife, artifacts from movements or from forced air warming therapy (Hemmerling and Migneault, 2002). Another EEG-based index is the Sample-entropy introduced by Richman et al. (Richman and Moorman, 2000). It is a variant of the approximate entropy that gives information on the complexity of a time series such as an EEG signal. Altogether then, although the EEG remains to these days the gold standard for the evaluation of the DoA it has some limitations and it is time consuming. Additional sensors would be useful. This may explain why, in routine clinical context, the best evaluation of the DoA is often deemed to be the one completed by the anesthesiologist. Therefore, a DoA monitor should ideally provide an evaluation without the help of the EEG signal.

Compulsory monitoring during the anesthesia includes heart rate, blood pressure, respiratory and muscle function, but it does not include the main target of the anesthetics i.e. the brain. To date, only a few teams aimed at evaluating the DoA using multiple parameters analysis. In 2014, Schneider et al. developed the Anesthesia Multimodal Index of Consciousness (AMIC) (Schneider et al., 2014). Their model included demographic data from the patients, the nature and quantity of the drugs injected and standard physiological signals such as heart rate, mean arterial blood pressure and EEG signals (Approximate entropy or Weighted spectral median frequency). It demonstrated a significantly better predictive value of the DoA compared to BIS values. In this study, the sampling rates for analysis of the physiological signals ranged between 10 seconds to 5 minutes. Their results are promising but we believe that increasing the sampling rate is critical in order to develop a real time approach. More recently, two studies have shown that artificial neuron networks fed with several standard parameters recorded during anesthesia could assess the DoA (Liu et al., 2015; Sadrawi et al., 2015). Sadrawi et al. used values sampled every 5-second on average (0.2 Hz), for both the EEG data and the physiologic parameters. In 2012, Liu et al. developed the University of Queensland Vital Signs Dataset (Liu et al., 2012), aimed at recording continuous monitoring values during anesthesia. Their recording was limited to 10 minutes, which does not allow proper recording of anesthesia procedures. Finally, databases have been used for a long time in anesthesiology but mainly to assess risks and improve safety in anesthesia practices.

Thus, there is a need for a system and method able to better predict the DoA in a real time approach compared to methods and systems described in the previous art.

## SUMMARY OF THE INVENTION

The invention is intended to remedy the disadvantages of the previous art. In the context of the invention, inventors used a specific protocol to monitor anesthetized patients in the operating room, with a commonly used monitoring device and they also recorded the EEG signal as ground truth to assess the level of anesthesia. Then, a stepwise protocol was used for data analysis and DoA prediction. Finally, a Hidden Markov Model (HMM) was trained on the recorded data from patient in order to predict and assess states based on four physiological variables that were adjusted to the consciousness level selected from Heart Rate (HR), Mean Blood Pressure (MeanBP), Respiratory Rate (RR) and the quantity of anesthetics agent. In particular, all these four physiological variables are used according to the invention, preferably without using EEG recordings.

The purpose of the invention is to assess the DoA in relation to intensive care, preferably in relation to a surgical operation and thus to secure it. Anesthesia is a medical procedure whose main objective is the temporary and reversible suspension of consciousness and painful sensitivity in order to perform a surgical operation in a patient. It can be general, loco-regional or local.

Indeed, for elderly or frail patients, establishing the optimal dosage of anesthetic agent may be critical. But sometimes, the BIS values are not well correlated with the stage of consciousness of the patient. More precisely, the estimated probability of good detection of LOC before this delay is less than a 50/50 chance and is equals to 0:455 (Laitio et al., 2008). Those technical constraints could lead to delay in taking decision and finally could have harming consequences on the most fragile patients. Moreover, as it will be described in example, transitions stages LOC and ROC were detected by the BIS with a 1 minute delay.

Hence, in a first aspect, the invention relates to a system for determining a representative value of depth of anesthesia of a patient comprising:

 a. acquisition means and communication means configured to receive data being generated from a patient from which a representative value of state of depth of anesthesia is to be determined,
 b. a storage module on which is recorded a trained Hidden Markov Model, said trained Hidden Markov Model having been trained in order to predict a value of depth of anesthesia with at least four physiological variables comprising: Heart Rate, Mean Blood Pressure, Respiratory Rate and quantity of anesthetics agent, and
 c. a data processing module connected to the storage module, said data processing module being configured for:

  i. Acquiring data of at least four physiological variables comprising a Heart Rate, a Mean Blood Pressure, a Respiratory Rate, and a quantity of anesthetics agent, said acquired data being generated from the patient from which a representative value of state of depth of anesthesia is to be determined;
  ii. Preprocessing acquired data to segment each physiological variable in several specific classes to reduce the inter-patient variability while keeping the intra-patient variability by mapping similar physiological states into same specific classes;
  iii. Loading the trained Hidden Markov Model; and
  iv. Determining a representative value of depth of anesthesia of the patient, selected from at least five states of the patient, said representative value corresponding to a highest probability calculated from the trained Hidden Markov Model and the preprocessed acquired data of said patient.

Advantageously, the trained Hidden Markov Model comprise the matrices involved in the model, namely the transition matrix, the belief matrix and the initial distribution. According to the invention, the term "matrice" refers to the hidden state of the HMM. Moreover, on the trained Hidden Markov Model, the state of depth of anesthesia has been inferred with EEG variables. In particular, the hidden states (corresponding to the state of depth of anesthesia) has been inferred with EEG variables preferably EEG data from at least one EEG channel recording either frontal or central lobe. Hence, a perfect segmentation of the time in the five states can be obtained. As the true segmentations are known, a conventional empirical measure can be used to learn the full Hidden Markov Model. In particular, the Hidden Markov Model has been trained with state of depth of anesthesia values inferred determined with EEG variables data recorded from the lobe position selected from Fp1-A2, Fp2-A1, F4-A1, F3-A2, C4-A1 and/or C3-A2. The use of such EEG data instead of BIS data improve the overall accuracy of the system and methods according to the invention.

Advantageously, the step of determining the representative value of depth of anesthesia comprise the use of a Viterbi algorithm. The Viterbi algorithm is used to predict the sequence of states of the patient, selected from Awake, Loss Of Consciousness (LOC), Anesthesia, Recovery Of Consciousness (ROC), and Emergence, said sequence of states of the patient corresponding to the highest probability calculated from the known sequence in the database, including all physiological data of the patient and of previous patients from recorded data and used to build the database and develop the algorithm.

Advantageously, the storage module is also configured to record, preferably continuously, data from patient having undergone anesthesia, said recorded data comprising at least Heart Rate, Mean Blood Pressure, Respiratory Rate, and quantity of anesthetics agent. Preferably, the storage module is configured to record data from patient having undergone anesthesia at a frequency of at least 1 Hertz.

Advantageously, the segmentation in step ii) is performed in three, four or five classes, preferably four, depending on the distribution of the acquired data. Such a segmentation in specific classes allow a reduction of the inter-patient variability while keeping the intra-patient variability by mapping similar physiological states into same specific classes.

Advantageously, the representative value of state of depth of anesthesia of the patient is determined without EEG variables as acquired data. Hence, the acquired data don't comprise EEG data. Indeed, the system according to the invention can determine a representative value of depth of anesthesia of the patient with a high degree of accuracy, superior to the BIS, and this without the need for the positioning of sensors necessary for the EEG on the patient.

However, in some embodiments, the data processing module is configured to acquire another physiological variable, by said variable is one EEG channel recording either from frontal or central lobe. Preferably, the one EEG channel is recorded by measuring mean from the lobe position selected from Fp1-A2, Fp2-A1, F4-A1, F3-A2, C4-A1 and/or C3-A2. These embodiments require the positioning of sensors necessary for the EEG on the patient but can enhance the accuracy of the determination.

Advantageously, the quantity of anesthetics agent is the quantity or concentration of the inspired anesthetics agent or the quantity or concentration of the expiratory concentration of anesthetics agent, preferably the quantity of anesthetics agent is the Expiratory Concentration of Anesthetics Agent.

Advantageously, the system of the present invention, in particular the acquisition means 10, continuously records at high frequency the physiologic parameters routinely monitored during anesthesia. In a preferred embodiment, the system is configured to record at a frequency of 1 Hertz. These double advantages (frequency of 1 Hz and recording of routine parameters) represent a critical difference compared to the available monitors.

In an embodiment of the invention the system is configured to acquire all data simultaneously or sequentially. Advantageously, the data acquired are synchronized.

In another preferred embodiment according to the invention, the system can include a 1-channel EEG (or one EEG channel) in order to build a database of properly labeled variables. The recording of one more physiological variable is advantageously located either frontal or central lobe, preferentially from the lobe position selected from Fp1-A2, Fp2-A1, F4-A1, F3-A2, C4-A1 and/or C3-A2, more preferentially from the Fp1-A2 lobe position. The implementation of the results of the single EEG channel in the algorithm is optional but as it may increase the precision of the DoA's assessment in certain cases, it is worth adding it as an optional measurement.

In a second aspect, the invention relates to a method for predicting depth of anesthesia of a patient to obtain a representative value of the depth of anesthesia of said patient, the method being carried out by a system according to the invention, said method comprising the steps of:

> i) Acquiring data of at least four physiological variables comprising a Heart Rate, a Mean Blood Pressure, a Respiratory Rate, and a quantity of anesthetics agent, from a patient from which a representative value of state of depth of anesthesia is to be determined
> ii) Preprocessing acquired data to segment each physiological variable in several specific classes to reduce the inter-patient variability while keeping the intra-patient variability by mapping similar physiological states into same specific classes;
> iii) Loading the trained Hidden Markov Model,
> iv) Determining a representative value of depth of anesthesia of the patient, selected from at least five states of the patient, said representative value corresponding to a highest probability calculated from the trained Hidden Markov Model and the preprocessed acquired data of said patient.

In an embodiment of the method of the invention, the dose of anesthetic agent is reduced to obtain the minimal effective dose to maintain the patient under surgical anesthesia according to the predicted depth of anesthesia.

In another aspect, the invention relates to a method to prevent over-dosage of anesthetic agent during the anesthesia of a patient, wherein the DoA is evaluated by the system according to invention.

In another aspect, the invention relates to a method to deliver a finely tuned anesthesia dose especially for frail patients, wherein the DoA is evaluated by the system according to invention.

The invention is particularly suited to predict the DoA of a patient to obtain a representative value of the DoA of said patient. Advantageously, the invention is suited to be used during surgery, preferentially surgery for patient at risk to develop complications during the procedure or after the administration of anesthetic agent such as elderly, frail patients or in case of organ failure such as cardiac or liver failure.

**LEGEND OF DRAWING**

Other advantages and characteristics of the disclosed system and method of the present invention will become apparent from reading the description, illustrated by the following figures, where:

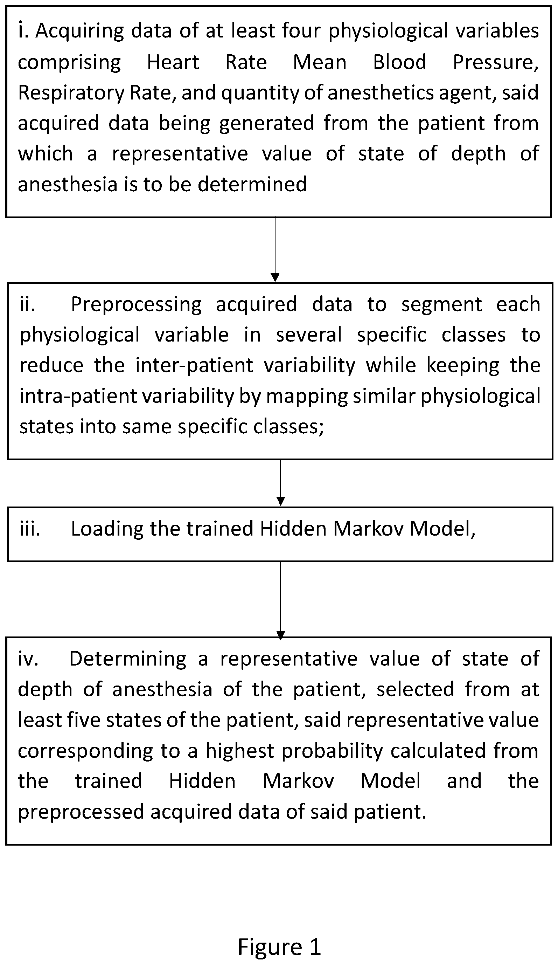

**Figure 1.** Flow diagram of the depth of anesthesia method according to the invention.

**Figure 2.** EEG signal of a patient during two different periods of time. On top 2A, the signal corresponds to an Awake patient while below 2B, it represents a signal during an Anesthesia state.

**Figure 3.** Typical spectrogram with anesthesia under sevoflurane. Units are seconds (s) for the time, Hertz (Hz) for the frequencies. The power in each band was then logarithmically transformed so that all spectral power was expressed in decibel (dB). The Loss Of Consciousness (LOC) stage is delimited by the two vertical line at the left of the spectrogram and the Recovery Of Consciousness (ROC) stage by the two vertical line at the right of the spectrogram.

**Figure 4.** Panel 4A represents a typical spectrogram during an anesthesia maintained by sevoflurane. Units are second (s) for time, Hertz (Hz) for frequency. The power in each band was then logarithmically transformed so that all spectral power was expressed in decibel (dB). Panel 4B represents BIS curve during anesthesia of the same patient.

**Figure 5.** Example of a discretization/segmentation on the two variables, HR (Heart Rate) and RR (respiratory rate). For each variable, on the top the raw signal recorded by the monitor during the GA. On the bottom, its discretization in four classes (0,1,2,3) via CKmean.

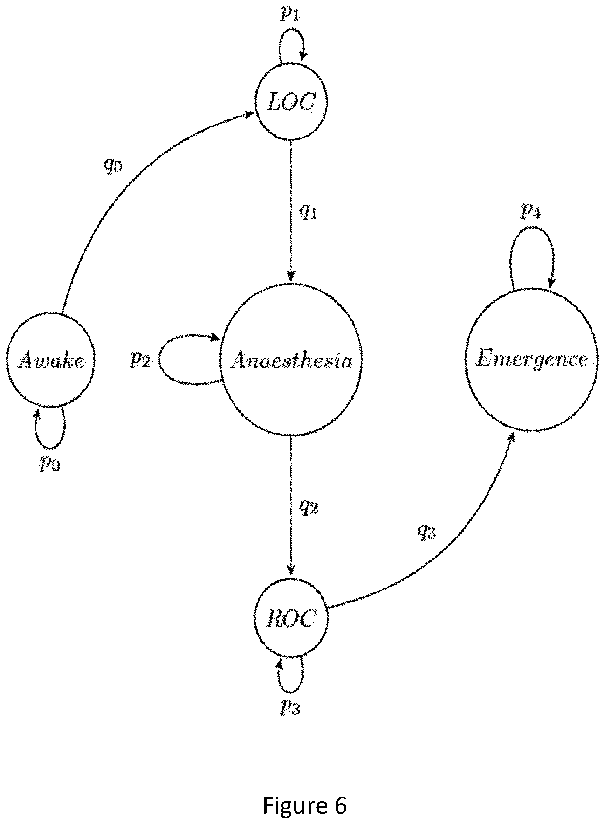

**Figure 6.** Diagram of the HMM. The 5 stages of the consciousness are represented. At each stage i there is a probability pi to stay in this stage or to go in another one qi. p0 refers to the probability to stay in stage Awake, p1 refers to the probability to stay in stage LOC, p2 refers to the probability to stay in stage Anesthesia, p3 refers to the probability to stay in stage ROC, p4 refers to the probability to stay in stage Emergence. q0 refers to the probability to go to stage LOC, q1 refers to the probability to go to stage Anesthesia, q2 refers to the probability to go to stage ROC, q3 refers to the probability to go to stage Emergence. The HMM model assess the values of (pi)i to predict the most probable sequence of stages of a patient during the surgery.

**Figure 7.** In full line the states of a patient during the surgery as assessed by the analysis of the EEG. In dotted line, the prediction of the HMM evaluated with the four aforementioned physiological variables (Heart Rate, Mean Blood Pressure, Respiratory Rate, and expired fraction of anesthetics agent).

**Figure 8** represents a system for predicting depth of anesthesia of a patient to obtain a representative value of said depth of anesthesia.

**DETAILED DESCRIPTION OF THE INVENTION**

As intended herein, the term "comprising" has the meaning of "including" or "containing", which means that when an object "comprises" one or several elements, other elements than those mentioned may also be included in the object. In contrast, when an object is said to "consist of" one or several elements, the object cannot include other elements than those mentioned.

One of the objectives in anesthesia is to prevent awareness without overdosing the patients with drugs. One of the achievements of anesthesia is the ability to monitor DoA. The overall incidence of intraoperative awareness with recall is about 0.2-3%, but it may be > 40% for high-risk patients, like, those with multiple trauma, caesarean section, cardiac surgery and hemodynamically unstable patients. Intraoperative awareness is a major medico-legal liability to the anesthesiologists and can lead to postoperative psychosomatic dysfunction in the patient, and therefore should be avoided at all costs.

According to the invention, the terms "subject", "individual", and "patient" are used interchangeably herein and refer to a mammal or several mammals in need of an anesthesia procedure. Subjects are preferably humans.

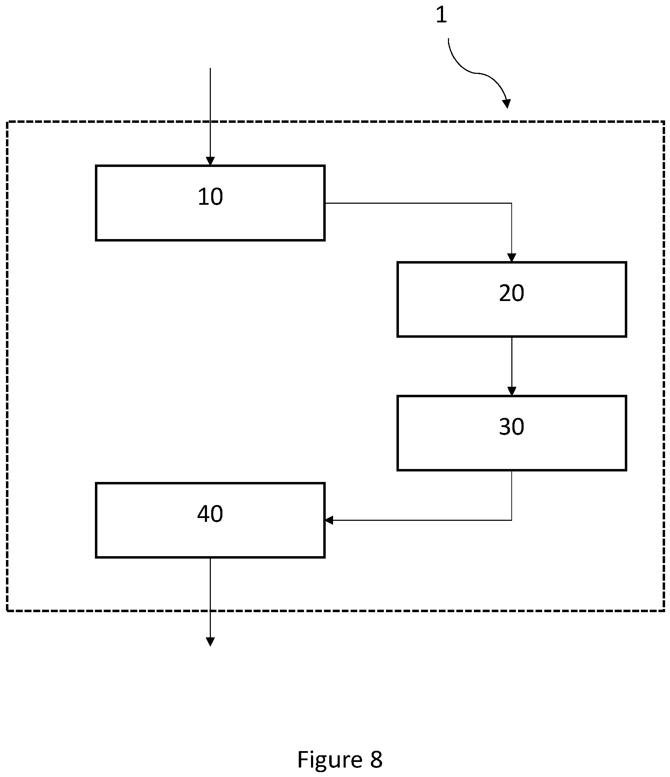

Thus, the present invention relates to a system 1 for determining a representative value of depth of anesthesia of a patient. Such a system 1, illustrated in figure 8 can comprise: acquisition means 10, communication means 20, a storage module 30, a data processing module 40. Preferably, a system 1 according to the invention comprise a storage module 30 and a data processing module 40.

The storage module 30 and the data processing module 40 are connected to communication means preferably configured to receive data being generated from a patient from which a representative value of state of depth of anesthesia is to be determined. Such data are preferably generated by acquisition means 10 which will be described later.

In a preferred embodiment, the data processing module 40 connected to the storage module 30 are configured for:

i. Acquiring data of at least four physiological variables comprising a Heart Rate, a Mean Blood Pressure, a Respiratory Rate, and a quantity of anesthetics agent delivered, from a plurality of patients,

ii. Acquiring data on value of known states of depth of anesthesia of patients corresponding to the previously acquired at least four physiological variables,

iii. Preprocessing said acquired data to segment each physiological variable in several specific classes,

iv. Training a Hidden Markov Model from preprocessed data and data on value of known states of depth of anesthesia, in order to predict a value of depth of anesthesia with at least four physiological variables comprising a Heart Rate, a Mean Blood Pressure, a Respiratory Rate and quantity of anesthetics agent.

Preferably said known states of depth of anesthesia have been determined using EEG data, more preferably data from at least one EEG channel recording either frontal or central lobe. Even more preferably, the Hidden Markov Model has been trained with states of depth of anesthesia inferred from EEG data recorded from the lobe position selected from Fp1-A2, Fp2-A1, F4-A1, F3-A2, C4-A1 and/or C3-A2.

Moreover, in a more preferred embodiment the data processing module 40 connected to the storage module 30 is configured for:

i. Acquiring data of at least four physiological variables comprising a Heart Rate, a Mean Blood Pressure, a Respiratory Rate, and a quantity of anesthetics agent, said acquired data being generated from the patient from which a representative value of state of depth of anesthesia is to be determined;

ii. Preprocessing acquired data to segment each physiological variable in several specific classes to reduce the inter-patient variability while keeping the intra-patient variability by mapping similar physiological states into same specific classes;

iii. Loading the trained Hidden Markov Model; and

iv. Determining a representative value of depth of anesthesia of the patient, selected from at least five states of the patient, said representative value corresponding to a highest probability calculated from the trained Hidden Markov Model and the preprocessed acquired data of said patient.

Advantageously, the step of determining the representative value of depth of anesthesia are determined by using Viterbi algorithm. Indeed, such Viterbi algorithm allow a fast processing of acquired data and permit the determination of a representative value of depth of anesthesia of the patient in real time.

According to the invention, "depth of anesthesia" relates to the probability of non-response to stimulation, calibrated against the strength of the stimulus, the difficulty of suppressing the response, and the drug-induced probability of non-responsiveness at defined effect site concentrations. Measurements are based on multiple different stimuli and responses at well-defined drug concentrations. There is no single stimulus and response measurement that will capture DoA in a clinically or scientifically meaningful manner. Hence the difficulties to measure, predict the DoA because it requires calibration of these observations of stimuli and responses (verbal responses, movement, tachycardia,...) against the dose and quantity of anesthetic drugs used to reduce the probability of response, constantly adjusting the administered dose to achieve the desired anesthetic depth. Due to interindividual variations (mainly enzymatic and metabolic variations), the perfect dose may vary from one patient to another even if they present the same comorbidities and apparent condition. Further, there are both objective and subjective methods for assessing DoA. Subjective methods comprise the evaluation of autonomic responses such as hemodynamic changes, lacrimation, sweating, pupillary dilatation, or isolated forearm technique. Objectives methods comprise spontaneous surface electromyogram, lower esophageal contractility, heart rate variability such as Bispectral index (BIS) and EEG. For example, the BIS is the most widely used anaesthetic depth device and method in both children and adults.

When patients require a general anesthesia, lots of physiological variables are recorded to monitor said patient, not only to monitor the DoA. According to the invention, the inventors proposes using at least four physiological variables such as Heart Rate, Mean Blood Pressure, Respiratory Rate, and quantity of anesthetics agent to build a database and further evaluate, predict the DoA using the system 1 and method of the invention.

Today, five states of consciousness are commonly used to evaluate the anesthesia process: Awake, Loss Of Consciousness (LOC), Anesthesia (meaning surgical anesthesia), Recovery Of Consciousness (ROC), and Emergence.

Awake is the state before induction of anesthesia begins. Loss Of consciousness (LOC) begins with induction and ends when the patient's lost consciousness. During this stage the patient presents a slow and regular breathing. From an ocular point of view, palpebral reflex is present. The patient may remain conscious and responsive to certain stimuli but the threshold of painful perception is diminished. Anesthesia is the state, when the surgery can take place, meaning that the patient is deemed to be totally unresponsive and consequently placed under mechanical ventilation. Recovery of Consciousness (ROC) starts when administration of the anesthesia drugs stopped and finished when the patient becomes responsive to simple order and has recovered a spontaneous breathing. State of Emergence can be defined as a state of consciousness of an individual when he is awake or easily arousable and aware of his surroundings and identity while clearing the anesthetic agents from the brain.

The inventors discovered that in order to obtain a reliable assessment of the DoA of a subject. A multiparametric method combining the analysis of several physiological variable from different variable families was essential. Among a multitude of physiological variable, the inventors have selected the 4 minimum physiological variables to correctly predict the DoA. They found that the system 1 and method must incorporate the process of at least Heart Rate variable, at least Mean Blood Pressure variable, at least Respiratory rate variable and at least quantity of anesthetics agent.

Thus, the physiological variable calculated in the method according to the invention include:

- at least one parameter relative to the Heart Rate,
- at least one parameter relative to the Blood Pressure,
- at least one parameter relative to the Respiratory rate, and
- at least one parameter relative to the quantity of anesthetics agent.

Thus, the system 1 can comprise acquisition means 10 able to measure at least four physiological variables of the patient. Said acquisition means are well known to the skilled person, for example said acquisition means relates to one acquisition mean for each physiological variable recorded. For example, acquisition mean for a Heart Rate can be heart rate monitor, acquisition mean for Blood Pressure can be a blood pressure sensor and allows rapid and simple measurement of systolic and diastolic blood pressure. Alternately, said heart rate sensor and blood pressure sensor is the same and can be recorded systolic and diastolic blood pressure as well as heart rate. Acquisition mean for Respiratory Rate can be Respiratory Rate sensor. In a preferred embodiment, one acquisition mean can be recorded all four physiological variables. Preferably, the Blood Pressure is the mean arterial Blood Pressure.

Further, the system 1 comprises a storage module 30 connected to the acquisition means on which are recorded from patient, said at least four physiological variables comprising a Heart Rate, Mean Blood Pressure, Respiratory Rate, and quantity of anesthetics agent to build a database. The storage module 30 where is recording all data can be all types of storage module 30 or memory such as transient or non-transient memories. This recording is preferably made on a non-transitional memory.

The storage module 30 could be connected to the acquisition means either wirelessly or with a wire connection. Advantageously, this a wire connection which is more adapted, secured than wirelessly connection for using in the surgical room. For example, USB connectivity.

In another embodiment, the data processing module 40 is connected to the storage module 30, for example, the data processing module 40 is a personal computer. Further, said data processing module 40 being configured for:

- Preprocessing the recording data to segment each physiological variable in several specific classes.
- Estimating the matrices involved in the Hidden Markov Model from the database.
- Using the Viterbi algorithm to predict the sequence of states of the patient, selected from Awake, Loss Of Consciousness (LOC), Anesthesia, Recovery Of Consciousness (ROC), and Emergence, said sequence of states of the patient corresponding to the highest probability calculated from the known sequence in the database, including all physiological data of the patient and of previous patients used to build the database.

To obtain a representative value which could be used to evaluate the DoA, a first step of preprocessing the recoding data is needed. Recording data are raw data obtained from the acquisition means, so the processing module 40 segment each physiological variable in several specific classes. Indeed, as each patient is unique, recorded data can vary greatly between to patient. Hence, recorded data need to be segmented in several classes dans each class will be used to determine the representative value of DoA.

Thresholds values for classes selection can be personalized between patient depending on other physiological data (age, BMI, ...). Indeed, a low value for a patient could be a high value for another patient. However, advantageously, the segmentation in step ii) is performed in three, four or five classes, preferably four, related to the distribution of the acquired data. Segmentation according to data distribution is a way of realizing segmentation that is highly appropriate for the patient.

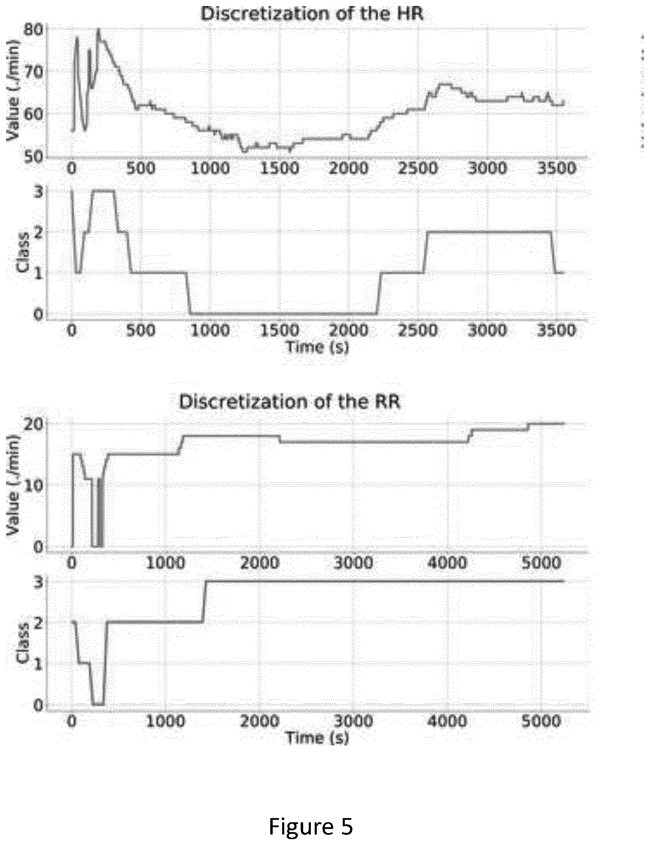

In detail, for each physiological variable, data obtained is segmented in three, four or five classes. For example, data obtained from Heart Rate are segmented in four different classes to correctly process and manage said data. This step of segmentation (or discretization) is necessary 1) to reduce the inter-patient variability while keeping the intra-patient variability by mapping similar physiological states into the same discretized state- a key part of the problem, as incoherent discretization led to contradictory events, 2) to train a model that automatically adapts to the demographic characteristics of patient (e.g. age, height, weight, BMI). An example of discretization is displayed in the Figure 5.

In particular, each observation consisted of quadruplets Heart Rate, Blood Pressure, Respiratory rate, quantity of anesthetics agent discretized using 3 thresholds. For example, this will lead to taking their values in a set {0, 1, 2, 3} - where 0 represents low values, and 3 high values. The discretization can be calculated using Ckmeans, a clustering algorithm based on K-means which has been proven to outperform it in the one-dimensional case.

In addition, segmentation and defined classes can be corrected over time because the more data are processed, the more the classification can be refined. Furthermore, segmentation can also be based, at least partially, on a predetermined classification according to the data of the studied patient. The use of such a predetermined classification can be of importance at the beginning of the process when only a small amount of data can be used to establish the classification.

Secondly, the data processing module 40 is configured to estimate matrices involved in the Hidden Markov Model from the database, more particularly from the processed segmented data by the data processing module 40 for each physiological variable and deleted intra-patient variability.

To learn the model, first, each entry of matrices is estimated using empirical distribution functions (more detailed in the following). The dataset involved in order to compute the empirical distribution functions is selected via a random split of the initial complete dataset (Train / Test).

Finally, the data processing module 40 is configured to predict the sequence of states of the patient, selected from Awake, Loss Of Consciousness (LOC), Anesthesia, Recovery Of Consciousness (ROC), and Emergence, said sequence of states of the patient corresponding to the highest probability calculated from the known sequences in the database, including all physiological data of the patient and of previous patients used to build the database.

A Markov chain is a tuple $(S, T, \pi)$ where $S$ is a finite set of states, $T$ is the transition matrix such as $T(s, \dot{s}) = P(S_{t+1} = s \vee S_t = \dot{s})$ and $\pi$ is the initial distribution. The main property of a Markov chain can be expressed as *"The future is independent of the past given the present"*. In mathematical term, $P(S_{t+1} = \dot{s} \vee S_t) = P(S_{t+1} = \dot{s} \vee S_t, ..., S_0)$. Thus, a HMM is a more complex Markov chain where states $S_t \in S$ cannot be directly observed. However, the model is supposed to provide observations carrying information about the states. Observations $O_i \in O$ give an assumption on those hidden states via a distribution $P(S_i \vee O_i)$ which is the probability of a state $S_i$ given a particular observation $O_i$. For instance, the probability to be in state Anesthesia if we observe all the variables equals to 0. Hence, a HMM is a tuple $(S, T, O, B, \pi)$ where $B$ is a stochastic matrix such as $B(s, x) = P(O_i = x \vee S_i = s)$. Observations were encoded with thresholds obtained by the calculation of the percentiles. This operation discretized the observations to construct the Belief matrix $B$.

Each entry of matrices is estimated using empirical distribution functions. As an example, the entry $(s, \dot{s})$ of the transition matrix $T$, $T(s, \dot{s}) = P(S_{t+1} = s \vee S_t = \dot{s})$, is estimated by the empirical distribution function i.e. by the number of time event s happen after even $\dot{s}$ in the dataset divided by all the possibility. When the elements HMM are learned (estimated), at a given time, the probability of a particular event given observations is calculated with the Viterbi algorithm.

In optional aspect of the invention, the data processing module 40 is configured to have an additional step of preprocessing to obtain optimal results. Each patient is unique and thresholds values needed to be personalized. For example, a low value for a patient could be a high value for another patient.

Optionally, the system comprises a monitor screen to display all the information about the physiological variable and the representative value of said DoA of the patient. Said representative value can be displayed, via an audible signal or visual signal. Visual signal can be a light, numbers, warning message, literal indication displaying Awake, Loss of Consciousness (LOC), Anesthesia, Recovery Of Consciousness (ROC) or Emergence. For example, the monitor screen is a touch-screen monitor.

In a preferred embodiment, the system 1 is configured to record data, in particular in the storage module 30, wherein data are recorded continuously. Preferably, the data recorded in the storage module 30 are recorded at least at a frequency of 1 Hertz. In their study, Schneider et al. used a sampling rates for analysis of the physiological signals that ranged between 10 seconds to 5 minutes. Sadrawi et al. used values sampled every 5-second on average (0.2 Hz), for both the EEG data and the physiologic parameters.

To the best of our knowledge, no other studies have been published using similar multi-modal methodology to assess the DoA. Increasing the frequency will allow a better and faster detection of eventual over or low dosage of anesthesia and is therefore a key problem in this field.

In an optional embodiment, the system 1 comprises one other physiological variable recorded in the storage module 30, said variable being one EEG channel recording either frontal or central lobe.

More particularly, the one EEG channels are recorded in the storage module 30 from the lobe position selected from Fp1-A2, Fp2-A1, F4-A1, F3-A2, C4-A1 and/or C3-A2, more particularly from the Fp1-A2 or Fp2-A1 position.

As described above, the system 1 comprises a storage module 30 connected to the acquisition means 10 which are recorded from patient. Further, at least four physiological variables comprising Heart Rate, Mean Blood Pressure, Respiratory Rate, and quantity of anesthetics agent are recorded to build a database. In a particular embodiment of the invention the quantity of anesthetics agent measured by measure means is the quantity of the Inspired Fraction of Anesthetics Agent.

Advantageously, all data recorded by the system 1 are recorded simultaneously or sequentially. Preferably, data recorded from each physiological variable, are synchronized to allow a precise correlation between the different parameters.

In a second aspect, the invention relates to a method for predicting depth of anesthesia of a patient to obtain a representative value of the depth of anesthesia of said patient, the method being carried out by a system 1 according to the invention, said method comprising the steps of:

    i. Acquiring data of at least four physiological variables comprising a Heart Rate, a Mean Blood Pressure, a Respiratory Rate, and a quantity of anesthetics agent delivered, from a patient from which a representative value of state of depth of anesthesia is to be determined

    ii. Preprocessing acquired data to segment each physiological variable in several specific classes;

    iii. Loading the trained Hidden Markov Model,

    iv. Determining a representative value of depth of anesthesia of the patient, selected from at least five states of the patient, said representative value corresponding to a highest probability calculated from the trained Hidden Markov Model and the preprocessed acquired data of said patient.

Advantageously, the step of acquiring data from the patient comprises one more physiological variable, said variable being one EEG channel recording either frontal or central lobe. In a preferred embodiment, the step (iv) determining a representative value of depth of anesthesia of the patient is realized with EEG.

The system according to the invention, wherein the one EEG channels are recorded in the lobe position in the group consisting of Fp1-A2, Fp2-A1, F4-A1, F3-A2, C4-A1 and/or C3-A2, more particularly in the Fp1-A2 or Fp2-A1 position.

In another aspect, the invention relates to a method to monitor the DoA and adapt the dosage of anesthetic agent, wherein the DoA is evaluated by the system 1 according to the invention and the dose of anesthetic agent is reduced to obtain the minimal effective dose to maintain the patient under surgical anesthesia.

In a preferred embodiment of a method according to the invention, a dose of anesthetic agent is reduced to obtain the minimal effective dose to maintain the patient under surgical anesthesia according to the predicted depth of anesthesia.

Further aspects and advantages of the invention will be disclosed in the following examples, which should be considered illustrative.

**EXAMPLE**

**MATERIEL & METHODS**

**2.1 Participants**

The study has been approved by the head of the institutional ethical review board of the French society of anesthesiology (SFAR). The patient criteria of inclusions were: 18 years of age minimum, inguinal hernia repair under general anesthesia, acceptance of the patient with low comorbidity score (patient classified ASA 1 or 2). The demographic data of the 30 patients are presented in table 1 below.

**2.2 Anesthesia protocol**

All patients were pre-oxygenated via face-mask by 100% oxygen for at least 3 minutes before induction. SUFENTANIL 0.3 ⍰ g/kg was injected rapidly followed, 3 minutes later, by 2-4 mg/kg of PROPOFOL in combination with KETAMINE 20 mg. When required for the surgery, patients were paralyzed following induction with a bolus of 0.17 mg/kg of CISATRACURIUM. After tracheal intubation, patients were ventilated with tidal volume of 6 mg/kg ideal-body weight, 5 cmH$_2$O Positive end-expiratory Pressure (Peep) and a respiratory rate between 10 and 14 to maintain EtCO$_2$ between 30 and 40 mmHg. Anesthesia was maintained with SEVOFLURANE MAC age-adjusted (e.g. 1.0). Dose adjustments were made by the anesthesiologist in charge of the patient depending on clinical variables available. Once asleep, patients received a transversus abdominis plane block with single bolus dose of ROPIVACAÏNE 150 mg.

Table 1: Demographic description of the population. The values presented are medians (minimums, maximums).

| Measures | Values |
|---|---|
| Age, yr | 60 (24, 92) |
| Sex F/M | 10/20 |
| Weight, kg | 82 (50, 105) |
| Height, cm | 176 (159, 191) |
| ASA I/II | 12/18 |

### 2.3 Equipments

#### 2.3.1 Hardware

**Monitoring device** - Each room in the intensive care unit or in the operating theatre is equipped with a multi parametric monitoring device called a scope and more precisely a Carescape monitor B850, from General Electrics (GE) Healthcare™ Finland Oy, Helsinki, Finland. The common monitoring includes Electrocardiogram (EKG), Arterial Pulse Oxymetry (SpO$_2$), Non-invasive or Invasive Arterial Blood Pressure. Optional monitoring offers more possibilities, such as gas analysis, EEG, plethysmography, BIS or Entropy. These variables can be monitored separately and displayed on a touch-screen monitor in real time.

#### 2.3.2 Software

**VSCapture** - A slighty modified software was used to record and save all the data presented in table 2 at the specified sampling frequencies imposed by the sensors specificities (Karippacheril and Ho, 2013). Importantly, all the recorded variables were synchronized within the GE scope, which allowed for a precise correlation between the different parameters.
**Python and Matlab™** - Libraries for preprocessing and signal analysis were: for Python- numpy, scipy, matplotlib. for Matlab™ - Signal Processing Toolbox.

### 2.4 Measurements

In order to perform multimodal analysis, the EKG and all the physiological variables routinely followed up for anesthesia (Table 2) were included in the recording. EEG was added to characterize objectively the state of consciousness (Harper, 1966; Samuels, 2007).

#### 2.4.1 Electroencephalogram (EEG)

EEG signal is the reference measurement for the monitoring of brain electrical. Inventors used a 2-channel EEG recorded at 100 Hz using the optional module E-EEG sold by GE™. Channels F4-A1 and C4-A1 were selected because of their ease of installation and their appropriate position on the scalp for anesthesia assessment. Indeed, several studies have demonstrated sustained modifications in the anterior part of the brain under general anesthesia. During a classical intervention involving sevoflurane, the frequencies of interest are below 30 Hz. For this reason, the sampling rate of the EEG - 100 Hz - was considered suitable for the present study.

#### 2.4.2 Physiological variables

Physiological variables were recorded at 1 Hz in their respective unit. During a general anesthesia for a low-risk patient, the 22 variables presented in table 2, were chosen as they included all the monitoring recommended by the ASA for anesthesia practice. Other variables were also recorded, such as ST-segment measurement, temperature and entropy. They are presented in Table 2.

**3 STEPWISE PROCEDURE**

**3.1 Preparation and Recording Steps**

**3.1 Materials needed**

- GE™ Carescape monitor B850
- Different modules added to the GE™ Carescape monitor to record the desirable variables as presented in Table 2.
- Four dry electrodes -two for the EEG recording, one for the mass and one for reference - connected to the EEG module.
- One computer with the VSCapture software installed. Note that because of C# architecture, the software can run on all OS (Linux, Windows or Macintosh).

Table 2: Recommended variables in the acquisition classified by modules.

| Variables | Units | abbreviation |
|---|---|---|
| **EKG - 300 Hz** | | |
| Electrocardiogram 1 | V | EKG |
| **E-EEG Module - 100 Hz** | | |
| Electroencephalogram 1 2 | V | EEG |
| **Basics Module -1 Hz** | | |
| Heart Rate | /min | HR |
| Systolic arterial blood pressure | mmHg | SBP |
| Diastolic arterial blood pressure | mmHg | DBP |
| Mean arterial blood pressure | mmHg | MBP |
| Saturated percentage of Dioxygen | /100% | $SpO_2$ |
| **Gas Analysis Module -1 Hz** | | |
| End tidal carbon dioxyde | mmHg | Et $CO_2$ |
| Anesthesia Agent | | AA |
| AA Expiratory quantity | /100% | AA ET |
| AA Inspiratory quantity | /100% | AA FI |
| Total Minimum Alveolar Concentration | /100% | AA MAC SUM |
| Fraction inspired of dioxygen | /m | $FiO_2$ |
| Mean alveolar concentration | /m | MAC |
| Fraction inspired Nitrous Oxide | /m | Fi $N_2$ |
| End tidal Nitrous Oxide | /m | Et $N_2O$ |
| Respiratory Rate | /min | RR |
| **BIS Module -1 Hz** | | |
| Bispectral Index | | BIS |
| BIS Burst Suppression Ratio | % | BIS BSR |
| BIS Electromyography | dB | BIS EMG |
| BIS Signal Quality Index | % | BIS SQI |

### 3.2 Sensors installation

For EEG recording, the scalp of each patient was cleaned with an abrasive cleaning fluid (Reegaponce pasta®; MEI, Toulon, France); low-impedance electrode pasta (Grass EC2 electrode cream®; Astro-Med, Warwick, Rhode Island, USA) was placed under a re-usable Capsulex electrode (Micromed Spa®, Mogliano Veneto, Italy). Electrodes impedance was kept under 2 kohm to ensure a proper EEG recording with minimal noise.

### 3.3 Connectivity

The connectivity used comprises four usb ports of the GE™ Carescape monitor B850 and are USB 2.0 ports. The computer was connected to the scope via a VGA/USB cable plugged on the monitor's second or third usb port. The first usb port is often used for the tactile keyboard and therefore should be avoided. The fourth usb port should also be avoided because its data transfer rate is smaller than for the three others.

### 3.4. Acquisition

Once VSCapture was started and the appropriate usb port was selected, recordings began automatically. Four.csv files were created: one for each EEG channel, one for the EKG channel and one for all the physiological variables. Another file, named "AS3Rawoutput1.raw" was automatically created but had no specific utility for our purpose and was deleted at the end of the recording. The first column of data displayed the time sampling and the other columns displayed the values of the recorded variables. All the files were collected in a folder called "Data", which was created at each launch.

### RESULTS

### 4.1 Identification of Depth of Anesthesia through Brain Activities

EEG has been widely studied and several tools are validated for its interpretation during anesthesia. In the signal processing landscape, Fourier theory is one of the most popular tool. Another one is the Wavelet theory. It is used for epilepsy detection and EEG classification can help quantifying brain activity. Temporal Series and auto-regressive processes could also provide solutions for an approximation of the frequency domain.

### 4.1.1 Stage of Consciousness

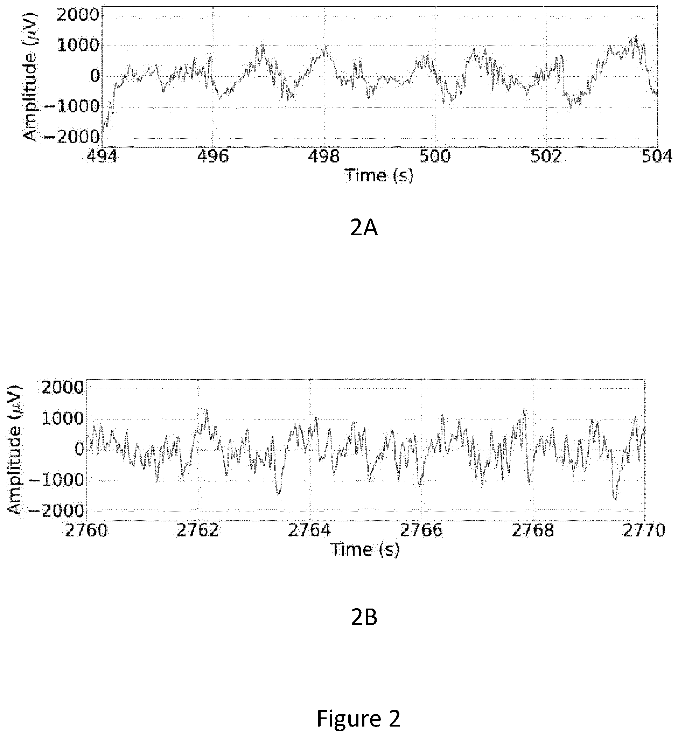

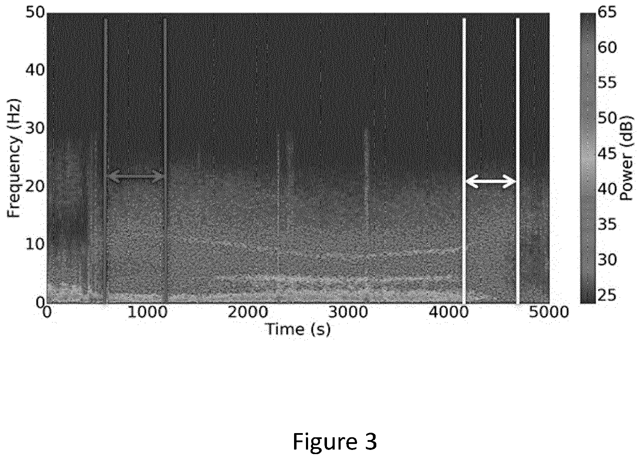

Despite huge progresses in anesthesia, particularly in the field of pharmacology, assessment of the DoA remains tricky. DoA is not directly observable and can only be estimated from the knowledge gained from various variables. The most robust way for DoA assessment in clinical practice is based on online EEG analysis, particularly the frontal channels. For instance, Bennett et al. (Bennett et al., 2009) showed that specific features of the EEG can be recognized and interpreted by anesthesiologists during a procedure. Indeed, extreme situations such as excessive brain activity or burst suppression state can be detected with minimal knowledge. It is similar to the clinical practice of EEG outside of the anesthesia field. Some major clinical abnormalities such as status epilepticus can be detected in real-time. But for other states (Loss Of Consciousness (LOC), surgical anesthesia, or Recovery Of Consciousness (ROC)) a precise determination is not realistic in clinical practice and remains the prerogative of research. Figures 2 and 3 show two different ways of analyzing an EEG during an anesthesia for a single patient. In figure 2, the upper part 2A presents the raw EEG of an awake patient, contrary to the lower part 2B where the patient is asleep. For example, the absolute value of the amplitude of the signal during the awake stage is lower than during the anesthesia stage.

### 4.1.2 EEG validation

EEG analysis should be performed by using appropriated mathematical tools based on time-frequency decomposition (see figure 2). This implies recognizing actual waves, which are presented in table 3.

Table 3: Definitions of waves with their names and frequencies.

| Waves | Frequencies (Hz) |
| --- | --- |
| $\delta$- Delta | 0 - 4 |
| $\theta$-Thêta | 4 - 8 |

(continued)

| Waves | Frequencies (Hz) |
|---|---|
| $\alpha$-Alpha | 8 - 14 |
| $\beta$-Bêta | > 14 |

Before any transformations and frequencies extraction of the signal we filtered it a 20Hz with a Butterworth bandpass filter of order 5 between 1 Hz and 20 Hz. This filtering removed the potential drift below 1 Hz, to keep the frequencies characterizing GA and to remove any noise over 20 Hz.

The spectrogram representation is certainly the easiest and fastest way to analyze an EEG and to present the data. Indeed, it is common to use it to visualize the variations in time and frequency of a signal. For instance, let s be a signal, sampled at frequency rate $f_s$ and composed of $N$ samples. Let $s[n]$ be the value of s at sample $n \in [\![ 0, N - 1 ]\!]$. Given a window size $N_w$, and a hop *size* $N_h$, we denote by $s^{(i)}$ the sub-signal of length $N_w$ and starting at sample $iN_h$, i.e.

$$s^{(i)} = s[iN_h : iN_h + N_w - 1].$$

The spectrogram representation is based on the calculation of the squared Discrete Fourier Transform (DFT) on the overlapping sub-signals $s^{(i)}$. It can be represented as a matrix with coefficients equal to

$$spectro\{s\}[i, k] = \left| \sum_{n=0}^{N_w - 1} w[n] s^{(i)}[n] e^{-2i\pi kn} \right|,$$

where $i$ is the frame number, $k$ is the frequency bin associated with physical frequency $f = \dfrac{k}{N_w} f_s$ and $w$ is a window of length $N_w$. This computation requires the choice of one window $w$, a window length $N_w$ and a hop size $N_h$.

In the spectrogram produced in Figure 3, each stage is easy to characterize. A classical window function called Hanning window or Hann window has been chosen. The window length $N_w$ was set to $2^{10} / 100 = 10.24$ seconds and the overlap to $N_h = N_w/2 = 5.12$ seconds.

The result of the analysis is a sequence, which defined in time three states during the surgery: the Loss Of Consciousness (LOC), the anesthesia and the Recovery Of Consciousness (ROC).

It allows supervised learning and supervised exploration for a better understanding of each stage.

The spectrogram of the same patient of figure 2 is displayed on figure 3. This is a typical recording of an unremarkable general anesthesia for inguinal hernial repair. Power values of interest varies from approximatively 35 to 60 dB. As expected, frequencies of interest are under 20 Hz and Band Frequencies are clearly identifiable in Figure 3. A visual analysis allows an extraction of the different stages over time. The LOC, referring to the transition from awake to anesthesia, starts at the beginning of the $\alpha$ wave and ends at the beginning of the $\theta$ wave. Conversely, the ROC started with the disappearance of the $\theta$ wave and finishes when the $\alpha$ band is no longer identifiable. The accurate and objective identification of the DoA was possible thanks to the determination of the waves on the spectrogram. The most important parameter are delays of transition which reflect the time for the different compartments of the brain to get into or out of deep anesthesia. It should be noticed that such clear spectrograms are possible because EEG was recorded with dedicated EEG electrodes and not extracted from the BIS sensors. This is not trivial as many researches are being performed with values derived from BIS sensor with a lower quality of signal.

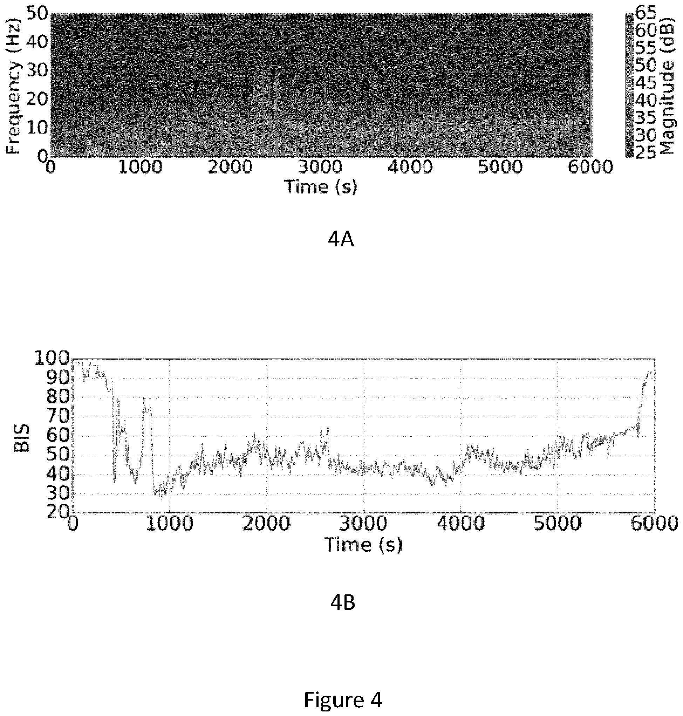

Spectrogram of an EEG signal with the same design than Figure 3 is plotted in Figure 4. Under 4B, the BIS curve was plotted to facilitate the comparison.

It is instructive to compare the BIS curve with our data. They often confirm the diagnosis of the stage determined by the EEG recordings. But sometimes, the BIS values are not well correlated with the stage of consciousness of the patient. It is the case at '900 seconds where values of the BIS became inexplicably high (Figure 4). Transitions stages LOC and ROC were detected by the BIS with a consequent delay -1 minute in our case-. More precisely, the estimated probability of good detection of LOC before this delay is less than a 50/50 chance and is equals to 0:455 (Laitio et al., 2008). Those technical constraints could lead to delay in taking decision and finally could have harming consequences on the most fragile patients.

**4.2 Electrocardiogram Segmentation**

**4.2.1 Automatic QRS Complex Detection**

The Electrocardiogram signal (EKG) is characterized by five mains waves referred as P, QRS complex (three waves) and T. Each one has a specific role during the cardiac cycle and their abnormalities will lead to different diagnoses or arrhythmia. To date, the gold-standard of EKG analysis remains human analysis, except in specific situations such as continuous ST-segment monitoring during anesthesia of high-risk cardiac patients. The PR interval is known to be linked to the autonomic nervous system. Drugs used during anesthesia are blocking the autonomous tone, explaining in a large part the side-effects of anesthesia. An automatic and real time detection of the PR interval appears potentially interesting. Inventors present some results of an automatic detection of QRS complex with a Pan and Tompkins filtering.

---

**Algorithm 1** Pan and Tompkins filtering

---

1: **Input :** filtered signal $\mathbf{s}[\cdot]$, threshold $\tau$, integer m
2: **Output :** signal $\mathbf{s}'$
3: $\mathbf{s} \longleftarrow$ Pass-Band filter on $\mathbf{s}$
4: **for** $i \in \{0,\ldots,\text{size}(\mathbf{s})-1\}$ **do**
5: $\quad \mathbf{s}_i \longleftarrow \mathbf{s}_i^2 - \mathbf{s}_{i+1}^2$
6: **end for**
7: **for** $i \in \{0,\ldots,\text{size}(\mathbf{s})-m\}$ **do**
8: $\quad \mathbf{s}_i \longleftarrow \dfrac{1}{m}\sum_{j=i}^{i+m}\mathbf{s}_j$
9: **end for**
10: $\mathbf{s}' \longleftarrow \mathbf{s}$
11: **for** $i \in \{0,\ldots,\text{size}(\mathbf{s})-1\}$ **do**
12: $\quad \mathbf{s}'_i \longleftarrow (\mathbf{s}_i < \tau)$
13: **end for**

---

**4.3 Anticipated results: Identification of Anesthesia State with Physiological Variables**

**4.3.1 Hidden Markov Model**

For a daily practice of anesthesia, an ideal DoA's monitor would provide a real-time evaluation without EEG and with a short-delay. The Hidden Markov chain used can be defined as shown in Figure 6. It contains the five classical states, Awake, LOC, Anesthesia, ROC and Emergence as hidden states and the probability to go from one state to another. In their model, inventors assume that transition only occurs between the current state and the next one. This was consistent with observations, since during the learning process, event "going back to previous state" was never identified. The interest of such a model is its ease for training and its simplicity.

Formally, a (finite-state) Markov chain is a tuple $(S, T, \pi)$ where $S$ is a finite set of states, $T$ is the transition matrix such as $T(s, \dot{s}) = P(S_{t+1} = sVS_t = \dot{s})$ and $\pi$ is the initial distribution. The main property of a Markov chain can be expressed as *"The future is independent of the past given the present"*. In mathematical term, $P(S_{t+1} = \dot{s} \text{ V } S_t) = P(S_{t+1} = \dot{s} \text{ V } S_t...,S_0)$. Thus, a HMM is a more complex Markov chain where states $S_t \in S$ cannot be directly observed. However, the model is supposed to provide observations carrying information about the states. Observations $O_i \in O$ give an assumption on those hidden states via a distribution $P(S_i \text{ V } O_i)$ which is the probability of a state $S_i$ given a particular observation $O_i$. For instance, the probability to be in state Anesthesia if we observe all the variables equals to 0. Hence, a HMM is a tuple $(S, T, O, B, \pi)$ where $B$ is a stochastic matrix such as $B(s, x) = P(O_i = x \text{ V } S_i = s)$. Observations were encoded with thresholds obtained by the calculation of the percentiles. This operation discretized the observations to construct the Belief matrix $B$. An additional preprocessing was performed for optimal results. As each patient was unique, thresholds values needed to be personalized. Indeed, a low value for a patient could be a high value for another patient. Note that, a tradeoff is necessary between the refinement of the model and the accuracy of the estimate probability.

**4.3.2 Results**

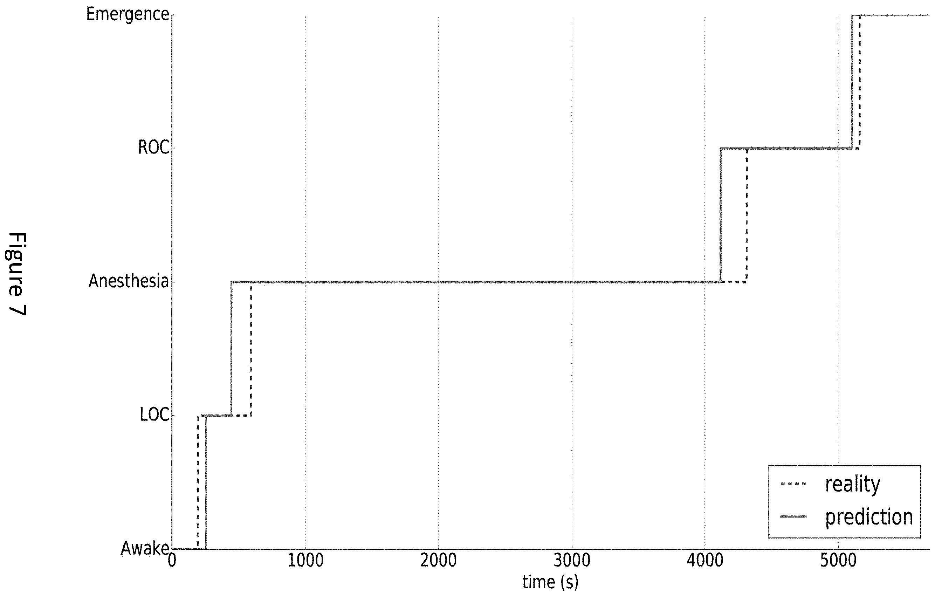

Figure 7 displayed the prediction of the model for one patient. The important information of this figure is the delay required to anticipate a change of state. One possibility to evaluate this model and its capacity to predict states on new patients is to use a leave-one-out technique. For each evaluation, one patient is put aside and the tuple of the HMM is learned on the others. The error of prediction is calculated on this single patient. Finally, the global error amounts to the average of all the previously computed errors. We used this algorithm on 16 patients under general anesthesia for hernial inguinal repair. The goal was to objectively identify the actual state among Awake, LOC, Anesthesia, ROC and Emergence. Each variable was divided as discussed above. The percentage of error for the prediction was 18%. The good performance obtained by our algorithm in a first group of 30 patients is promising for the future. Moreover, results with only three physiological variables have led to lower prediction quality compared to the use of a trained HMM with the four physiological variables: Heart Rate, a Mean Blood Pressure, a Respiratory Rate and anesthetics agent.

## 5. DISCUSSION

Inventors proposed here a full protocol for high-resolution monitoring of the anesthetized patient in order to predict the DoA. The three key practices in this work overlapped: the setting of sensors, the installation of all the devices and a solution to extract relevant information from the data. The confrontation with already published data proved the high quality of the recordings.

This constitutes the very first step on the way towards a multimodal approach of monitoring of general anesthesia.

## Conclusion

The fact that our first classifier already demonstrated a good predictability is very encouraging for the future. It is reasonable to imagine that results on a wider population will give better results. Predicting and ensuring an acceptable level of anesthesia could permit to decrease the anesthesia dose to the strict minimum required and therefore decrease the risk of complications. Another exciting field of research with multimodal parameters is the intensive care unit where patients may present more complex disease.

Avidan MS, Zhang L, Burnside BA, Finkel KJ, Searleman AC, Selvidge JA, et al. Anesthesia awareness and the bispectral index. N Engl J Med. 2008;358:1097-108.

Bennett C, Voss U, Barnard JP, Sleigh JW. Practical use of the raw electroencephalogram waveform during general anesthesia: the art and science. Anesth Analg. 2009;109:539-550.

Bruhn J, Myles PS, Sneyd R, Struys MM. Depth of anaesthesia monitoring: what's available, what's validated and what's next? Br J Anaesth. 2006;97:85-94.

George Mychaskiw I, Horowitz M, Sachdev V, Heath BJ. Explicit intraoperative recall at a bispectral index of 47. Anesth Analg. 2001;92:808-809.

Hemmerling TM, Migneault B. Falsely increased bispectral index during endoscopic shoulder surgery attributed to interferences with the endoscopic shaver device. Anesth Analg. 2002;95:1678-1679.

Karippacheril JG, Ho TY. Data acquisition from S/5 GE Datex anesthesia monitor using VSCapture: An open source. NET/Mono tool. J Anaesthesiol Clin Pharmacol. 2013;29:423.

Kissin I. Depth of anesthesia and bispectral index monitoring. Anesth Analg. 2000;90:1114-1117.

Laitio RM, Kaskinoro K, Särkelä MO, Kaisti KK, Salmi E, Maksimow A, et al. Bispectral index, entropy, and quantitative electroencephalogram during single-agent xenon anesthesia. J Am Soc Anesthesiol. 2008;108:63-70.

Liu D, Gorges M, Jenkins SA. University of Queensland vital signs dataset: Development of an accessible repository of anesthesia patient monitoring data for research. Anesth Analg. 2012;114:584-589.

Liu Q, Chen Y-F, Fan S-Z, Abbod MF, Shieh J-S. EEG signals analysis using multiscale entropy for depth of anesthesia monitoring during surgery through artificial neural networks. Comput Math Methods Med. 2015;2015.

Richman JS, Moorman JR. Physiological time-series analysis using approximate entropy and sample entropy. Am J Physiol-Heart Circ Physiol. 2000;278:H2039-H2049.

Schneider G, Jordan D, Schwarz G, Bischoff P, Kalkman CJ, Kuppe H, et al. Monitoring depth of anesthesia utilizing a combination of electroencephalographic and standard measures. J Am Soc Anesthesiol. 2014;120:819-828.

Sadrawi M, Fan S-Z, Abbod MF, Jen K-K, Shieh J-S. Computational depth of anesthesia via multiple vital signs based on artificial neural networks. BioMed Res Int. 2015;2015.

Whitlock EL, Villafranca AJ, Lin N, Palanca BJ, Jacobsohn E, Finkel KJ, et al. Relationship between Bispectral Index Values and Volatile Anesthetic Concentrations during the Maintenance Phase of Anesthesia in the B-Unaware Trial. Anesthesiology [Internet]. 2011; Available from: http://www.ncbi.nlm.nih.gov/entrez/query.fcgi?cmd=Retrieve&db=PubMed&dopt=Citation&list_uid s=22037642

**Claims**

1. A system (1) for determining a representative value of depth of anesthesia of a patient comprising:

   a. acquisition means (10) and communication means (20) configured to receive data being generated from a patient from which a representative value of state of depth of anesthesia is to be determined,
   b. a storage module (30) on which is recorded a trained Hidden Markov Model, said trained Hidden Markov Model having been trained in order to predict a value of state of depth of anesthesia with at least four physiological variables comprising: Heart Rate, Mean Blood Pressure, Respiratory Rate and quantity of anesthetics agent, and
   c. a data processing module (40) connected to the storage module (30), said data processing module (40) being configured for:

      i. Acquiring data of at least four physiological variables comprising Heart Rate Mean Blood Pressure, Respiratory Rate, and quantity of anesthetics agent, said acquired data being generated from the patient from which a representative value of state of depth of anesthesia is to be determined;
      ii. Preprocessing acquired data to segment each physiological variable in several specific classes to reduce the inter-patient variability while keeping the intra-patient variability by mapping similar physiological states into same specific classes;
      iii. Loading the trained Hidden Markov Model; and
      iv. Determining a representative value of state of depth of anesthesia of the patient, selected from at least five states of the patient, said representative value corresponding to a highest probability calculated from the trained Hidden Markov Model and the preprocessed acquired data of said patient.

2. The system (1) according to claim 1, wherein, on the trained Hidden Markov Model, the states of depth of anesthesia have been inferred with EEG variables.

3. The system (1) according to claim 1 or 2, wherein the Hidden Markov Model has been trained with state of depth of anesthesia values inferred with EEG variables recorded from the lobe position selected from Fp1-A2, Fp2-A1, F4-A1, F3-A2, C4-A1 and/or C3-A2.

4. The system (1) according to any of claims 1 to 3, wherein determining the representative value of depth of anesthesia comprise the use of a Viterbi algorithm.

5. The system (1) according to any of claims 1 to 4, wherein the storage module (30) is also configured to record, preferably continuously, data from patient having undergone anesthesia, said recorded data comprising at least Heart Rate, Mean Blood Pressure, Respiratory Rate, and quantity of anesthetics agent.

6. The system (1) according to claim 5, wherein the storage module (30) is configured to record data from patient having undergone anesthesia at a frequency of at least 1 Hertz.

7. The system (1) according to any of claims 1 to 6, wherein the segmentation in step ii) is performed in three, four or five classes, preferably four, depending on the distribution of the acquired data.

8. The system (1) according to any of claims 1 to 7, wherein the representative value of state of depth of anesthesia of the patient is determined without EEG variables as acquired data.

9. The system (1) according to any of claims 1 to 7, wherein the data processing module (40) is configured to acquire another physiological variable, by said variable is one EEG channel recording either from frontal or central lobe.

10. The system (1) according to claim 9, wherein the one EEG channel is recorded by measuring mean from the lobe position selected from Fp1-A2, Fp2-A1, F4-A1, F3-A2, C4-A1 and/or C3-A2.

11. The system (1) according to any of claims 1 to 10, wherein the quantity of anesthetics agent is the quantity or concentration of the inspired anesthetics agent or the quantity or concentration of the expiratory concentration of anesthetics agent, preferably the quantity of anesthetics agent is the Expiratory Concentration of Anesthetics Agent.

12. The system (1) according to any of claims 1 to 11, wherein all data are acquired simultaneously or sequentially.

13. The system (1) according to any of claims 1 to 12, wherein data acquired are synchronized.

14. A method for predicting depth of anesthesia of a patient to obtain a representative value of the depth of anesthesia of said patient, the method being carried out by a system (1) according to any one of claims 1 to 13, said method comprising the steps of:

> i. Acquiring data of at least four physiological variables comprising a Heart Rate, a Mean Blood Pressure, a Respiratory Rate, and a quantity of anesthetics agent, from a patient from which a representative value of state of depth of anesthesia is to be determined
> ii. Preprocessing acquired data to segment each physiological variable in several specific classes to reduce the inter-patient variability while keeping the intra-patient variability by mapping similar physiological states into same specific classes;
> iii. Loading the trained Hidden Markov Model,
> iv. Determining a representative value of depth of anesthesia of the patient, selected from at least five states of the patient, said representative value corresponding to a highest probability calculated from the trained Hidden Markov Model and the preprocessed acquired data of said patient.

15. The method according to claim 14, wherein the segmentation in step ii) is performed in three, four or five classes, preferably four classes, depending on the distribution of the acquired data.

16. The method according to claims 14 or 15, wherein the quantity of anesthetics agent is the quantity or concentration of the inspired anesthetics agent or the quantity or concentration of the expiratory concentration of anesthetics agent, preferably the quantity of anesthetics agent is the Expiratory Concentration of Anesthetics Agent.

17. The method according to any of claims 14 to 16, wherein a dose of anesthetic agent is reduced to obtain the minimal effective dose to maintain the patient under surgical anesthesia according to the predicted depth of anesthesia.

i. Acquiring data of at least four physiological variables comprising Heart Rate Mean Blood Pressure, Respiratory Rate, and quantity of anesthetics agent, said acquired data being generated from the patient from which a representative value of state of depth of anesthesia is to be determined

ii. Preprocessing acquired data to segment each physiological variable in several specific classes to reduce the inter-patient variability while keeping the intra-patient variability by mapping similar physiological states into same specific classes;

iii. Loading the trained Hidden Markov Model,

iv. Determining a representative value of state of depth of anesthesia of the patient, selected from at least five states of the patient, said representative value corresponding to a highest probability calculated from the trained Hidden Markov Model and the preprocessed acquired data of said patient.

Figure 1

2A

2B

Figure 2

Figure 3

4A

4B

Figure 4

Figure 5

Figure 6

Figure 7

Figure 8

Europäisches
Patentamt

European
Patent Office

Office européen
des brevets

## EUROPEAN SEARCH REPORT

Application Number

EP 19 30 6597

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/042475 A1 (VERGHESE GEORGE CHEERAN [US] ET AL) 16 February 2017 (2017-02-16) * paragraphs [0096], [0101] - [0105] * ----- | 1-16 | INV. A61B5/021 A61B5/024 A61B5/08 A61M16/10 |
| | | | TECHNICAL FIELDS SEARCHED (IPC) A61B A61M |

The present search report has been drawn up for all claims

| Place of search | Date of completion of the search | Examiner |
|---|---|---|
| Berlin | 9 June 2020 | Clevorn, Jens |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.........................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 30 6597

This annex lists the patent family members relating to the patent documents cited in the above-mentioned European search report.
The members are as contained in the European Patent Office EDP file on
The European Patent Office is in no way liable for these particulars which are merely given for the purpose of information.

09-06-2020

| Patent document cited in search report | Publication date | Patent family member(s) | Publication date |
|---|---|---|---|
| US 2017042475 A1 | 16-02-2017 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AVIDAN MS ; ZHANG L ; BURNSIDE BA ; FINKEL KJ ; SEARLEMAN AC ; SELVIDGE JA et al.** Anesthesia awareness and the bispectral index. *N Engl J Med.,* 2008, vol. 358, 1097-108
- **BENNETT C ; VOSS U ; BARNARD JP ; SLEIGH JW.** Practical use of the raw electroencephalogram waveform during general anesthesia: the art and science. *Anesth Analg.,* 2009, vol. 109, 539-550
- **BRUHN J ; MYLES PS ; SNEYD R ; STRUYS MM.** Depth of anaesthesia monitoring: what's available, what's validated and what's next?. *Br J Anaesth,* 2006, vol. 97, 85-94
- **GEORGE MYCHASKIW I ; HOROWITZ M ; SACH-DEV V ; HEATH BJ.** Explicit intraoperative recall at a bispectral index of 47. *Anesth Analg.,* 2001, vol. 92, 808-809
- **HEMMERLING TM ; MIGNEAULT B.** Falsely increased bispectral index during endoscopic shoulder surgery attributed to interferences with the endoscopic shaver device. *Anesth Analg.,* 2002, vol. 95, 1678-1679
- **KARIPPACHERIL JG ; HO TY.** Data acquisition from S/5 GE Datex anesthesia monitor using VSCapture: An open source. NET/Mono tool. *J Anaesthesiol Clin Pharmacol.,* 2013, vol. 29, 423
- **KISSIN I.** Depth of anesthesia and bispectral index monitoring. *Anesth Analg.,* 2000, vol. 90, 1114-1117
- **LAITIO RM ; KASKINORO K ; SÄRKELÄ MO ; KAISTI KK ; SALMI E ; MAKSIMOW A et al.** Bispectral index, entropy, and quantitative electroencephalogram during single-agent xenon anesthesia. *J Am Soc Anesthesiol.,* 2008, vol. 108, 63-70
- **LIU D ; GORGES M ; JENKINS SA.** University of Queensland vital signs dataset: Development of an accessible repository of anesthesia patient monitoring data for research. *Anesth Analg.,* 2012, vol. 114, 584-589
- **LIU Q ; CHEN Y-F ; FAN S-Z ; ABBOD MF ; SHIEH J-S.** EEG signals analysis using multiscale entropy for depth of anesthesia monitoring during surgery through artificial neural networks. *Comput Math Methods Med.,* 2015, 2015
- **RICHMAN JS ; MOORMAN JR.** Physiological time-series analysis using approximate entropy and sample entropy. *Am J Physiol-Heart Circ Physiol.,* 2000, vol. 278, H2039-H2049
- **SCHNEIDER G ; JORDAN D ; SCHWARZ G ; BISCHOFF P ; KALKMAN CJ ; KUPPE H et al.** Monitoring depth of anesthesia utilizing a combination of electroencephalographic and standard measures. *J Am Soc Anesthesiol,* 2014, vol. 120, 819-828
- **SADRAWI M ; FAN S-Z ; ABBOD MF ; JEN K-K ; SHIEH J-S.** Computational depth of anesthesia via multiple vital signs based on artificial neural networks. *BioMed Res Int.,* 2015, 2015
- **WHITLOCK EL ; VILLAFRANCA AJ ; LIN N ; PALANCA BJ ; JACOBSOHN E ; FINKEL KJ et al.** Relationship between Bispectral Index Values and Volatile Anesthetic Concentrations during the Maintenance Phase of Anesthesia in the B-Unaware Trial. *Anesthesiology [Internet,* 2011, http://www.ncbi.nlm.nih.gov/entrez/query.fcgi?cmd=Retrieve&db=PubMed&dopt=Citation&list_uids=22037642